# EUROPÄISCHE PATENTSCHRIFT

(11) **EP 0 977 653 B1**
(45) Veröffentlichungstag und Bekanntmachung des Hinweises auf die Patenterteilung: **04.06.2003**
(21) Anmeldenummer: 98924173.2
(22) Anmeldetag: 22.04.1998
(51) Int. Cl.: B25F 3/00, G01N 3/42

(54) **Bohrwiderstandsmessgerät zur Ermittlung des inneren Zustands von Bäumen oder Holzbauteilen**
Drill resistance measuring apparatus for determining the internal state of trees or wooden structures
Dispositif de mesure de la résistance de perçage pour déterminer l'état de l'intérieur d'un arbre ou d'un élément de construction en bois

(30) Priorität: 23.04.1997 DE 29707307 U
(43) Veröffentlichungstag der Anmeldung: 09.02.2000
(73) Patentinhaber: Forschungszentrum Karlsruhe GmbH, 76133 Karlsruhe (DE)
(72) Erfinder: MATTHECK, Claus, D-76774 Leimersheim (DE); HUNGER, Erich, D-69168 Wiesloch (DE)
(86) Internationale Anmeldenummer: EP9802385
(87) Internationale Veröffentlichungsnummer: WO98047671

(56) Entgegenhaltungen:
- EP-A- 0 673 723
- DE-A- 3 501 841
- DE-A- 4 004 242
- DE-A- 4 122 494
- DE-A- 4 438 383
- FR-A- 2 600 772
- GB-A- 2 242 029

## Beschreibung

Die Erfindung betrifft ein Bohrwiderstandsmessgerät bestehend aus einem Bohraufsatz und einem Antriebsaggregat nach dem Oberbegriff des Patentanspruchs 1, wie er aus der DE-A-44 38 383 bekannt ist.

Bei einem an und für sich bekannten Verfahren (DE-C-35 01841) werden an einem langen nadelförmigen Bohrer die zu untersuchenden, überwiegend aus Holz bestehenden Objekte angebohrt und dabei der Eindringungswiderstand gemessen, aufgezeichnet und/oder elektronisch gespeichert. Aus dem so ermittelten Widerstandsdiagramm lassen sich Rückschlüsse auf die innere Struktur des Versuchsobjektes ziehen, die dendrochronologischen Zwecken, der Ermittlung von Zuwachsraten, Auswirkungen von Krankheitsbefall und zur Überprüfung der Tragfähigkeit dienen.

Die bisher bekanntgewordenen, von elektrischen Batterien betriebenen Bohrgeräte besitzen einen auf einem Schlitten entlang einer Gleitbahn verschiebbaren Bohrmotor, dessen Vor- und Rückwärtsbewegung durch einen Vorschubmotor über einen Spindel- oder Zahnradantrieb bewirkt wird. Bekannt ist auch ein Vortrieb geworden, bei dem der Vorschubmotor mit auf dem Schlitten des Bohrmotors angeordnet ist und sich über ein mit einer festangeordneten Zahnstange kämmendes Ritzel fortbewegt.

Ehe die lange Bohrnadel bis zu einer gewissen Tiefe in das zu untersuchende Objekt eingedrungen ist, muß sie abgestützt werden, weil sie sonst unter der Einwirkung der Vorschubkraft zu einem Schwingbauch ausschlagen würde; als Abstützelemente finden dünne Rohre Verwendung, die sich entsprechend dem Vorschub teleskopisch ineinander schieben.

Manche Gerätetypen sind mit einer Aufzeichnungsvorrichtung ausgerüstet, um die Bohrung schon während ihrer Durchführung kontrollieren zu können. Die registrierende Schreib- oder Aufzeichnungsvorrichtung kann innen oder außen am Gehäusemantel angeordnet sein. Die Stromzufuhr erfolgt von einer externen Batterie über Kabel, es sind aber auch Geräte bekannt geworden, wo die Batterie am Gehäuse des Bohrgerätes befestigt ist. Ausnahmslos bestehen die bekannten Bohrgeräte aus einem rohrförmigen Schutzmantel eckigen oder runden Querschnitts, an dessen vorderen Ende sich der Bohrnadelaustritt, am hinteren Ende ein Handgriff mit Schalt-, Steck- und Steuerungselementen und im Rohr selbst die Führungsbahn und die Nadelabstützelemente befinden.

Die Bohrwiderstandsmessung für Baum- und Holzuntersuchungen hat sich seit ihrem Bekanntwerden weit verbreitet. Nachteilig für eine schnellere Verbreitung ist der relativ hohe Preis für die Untersuchungsgeräte, der wiederum durch die geringe Stückzahl an benötigten Geräten bedingt ist, weil sich eine verbilligte Massenfertigung nicht lohnt.

Die beschriebenen, für die Durchführung des Verfahrens notwendigen Elemente bedingen natürlich eine gewisse räumliche Ausdehnung in allen drei Richtungen und bedingen auch ein bestimmtes, relativ hohes Gewicht.

Da Bohruntersuchungen in vielen Fällen von schwer zugänglichen Stellen aus durchzuführen sind, wie es beispielsweise im Geäst von Bäumen, an hohen Decken in Gebäuden von Leitern aus, oder in verwinkelten Dachgestühlen der Fall ist, muß das Gerät sehr oft sogar in Überkopfhaltung von Hand geführt werden, dabei ist zur Vermeidung von Fehlern jedes Verkanten oder Abweichen von der Bohrrichtung zu vermeiden.

Des weiteren ist aus der DE-A-41 22 494 ein Bohrwiderstandsmeßgerät bekannt welches alle gattungsgemäßen Merkmale aufweist, wobei ein Planetengetriebe nicht als Kraftaufnehmer dienen kann.

Aufgabe der Erfindung ist es, ein Bohrwiderstandsmeßgerät möglichst kleiner räumlicher Ausmaße und möglichst geringem Gewicht zu schaffen, das eine leichte und zugleich sichere Handhabung ermöglicht und das kostengünstig gefertigt werden kann.

Gelöst wird diese Aufgabe durch die Merkmale des Patentanspruchs 1. Die abhängigen Ansprüche 2-12 beschreiben vorteilhafte Ausgestaltungen der Erfindung.

Die Geräte für die Bohrwiderstandsmessung haben in ihrem Grundprinzip eine ganze Reihe von Analogien mit elektrischen Handbohrmaschinen. Es ist deshalb auch schon der Vorschlag gemacht worden, grundsätzlich eine solche, - im Handel sehr billig im Vergleich zu den reinen Bohrwiderstands-Meßgeräten - als Grundgerät zu verwenden und mit einem Zusatzgerät für die besondere Aufgabenstellung auszurüsten, wie man es etwa von Zusatzgeräten kennt, die als Pumpen, Schleifmaschinen, Stichsägen, Schraubendreher o. a. arbeiten.

Ein so kurz gebautes Gerät ist nicht nur leicht zu handhaben, es besitzt zwischen Ansatz am Untersuchungsobjekt und Handgriff einen so kurzen Hebelarm, daß sich Verkantungen oder Bewegungen beim Bohrvorgang nicht oder nur geringfügig schädigend auswirken.

Die kurze Bauweise wurde durch die Kombination mehrerer Prinzipien und Konstruktionsmerkmale erreicht. So wurde im Gegensatz zu den auf dem Markt befindlichen Geräten neben dem natürlich erforderlichen Bohrmotor auf einen eigenen Vorschubmotor verzichtet und der Vorschub durch geeignete, - nachstehend detailliert beschriebene - Konstruktionsmerkmale von der Handbohrmaschine bewerkstelligt.

Ein bekannt gewordener Vorschlag (DE-A-40 04 242) sieht zwar auch schon vor, das Bohren und Vorschub von nur einem Motor aus durchführen zu lassen, doch wird außer der - selbstverständlichen - Angabe, daß Untersetzungsgetriebe zu verwenden sind, keine Konstruktionslösung aufgezeigt. Eine solche wäre für den genannten Vorschlag auch einfach, da der Bohrmotor auf dem Vorschubschlitten angeordnet ist, den er über einen zweiten Wellenzapfen selbst antreiben kann.

Die vorliegende Erfindung bewältigt sowohl das Bohren wie den Vorschub von einem außerhalb des eigentlichen Bohraggregates, nämlich den in der Handbohrmaschine liegenden Motor, wobei die Energieübertragung sowohl für das Bohren wie für den Vorschub auf nur einem mechanisch wirkenden Weg erfolgt.

Da es somit im eigentlichen Bohraggregat keine Motoren, Schalter oder Sensoren gibt, entfallen in ihm jegliche elektrische Installationen und Leitungen, Schleppkabel, Stromschienen, Schalter und Stecker, was in der Herstellung und der Montage eingespart werden kann.

Die Erfindung wird im folgenden anhand eines Ausführungsbeispiels mit Hilfe der Figuren näher erläutert.

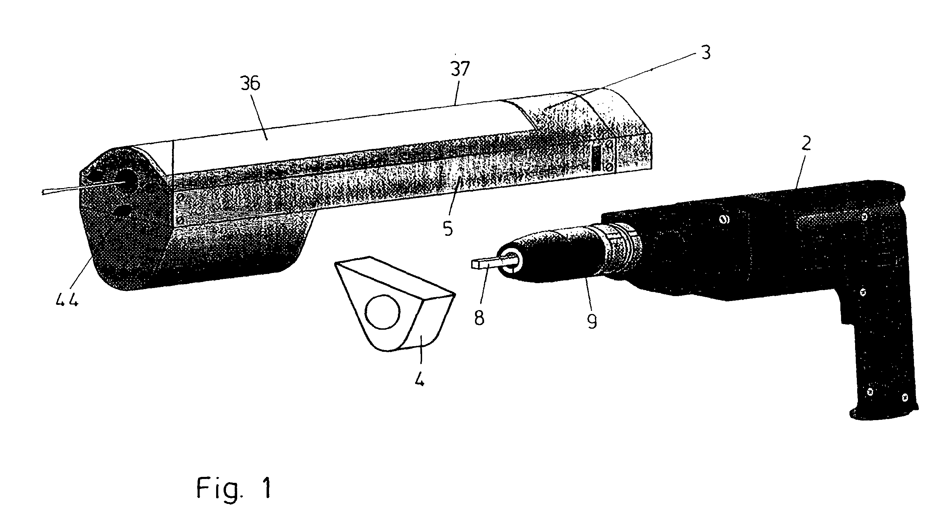

Dabei zeigt die Fig. 1 eine handelsübliche Bohrmaschine als Antriebsaggregat mit dem Bohraufsatz und die

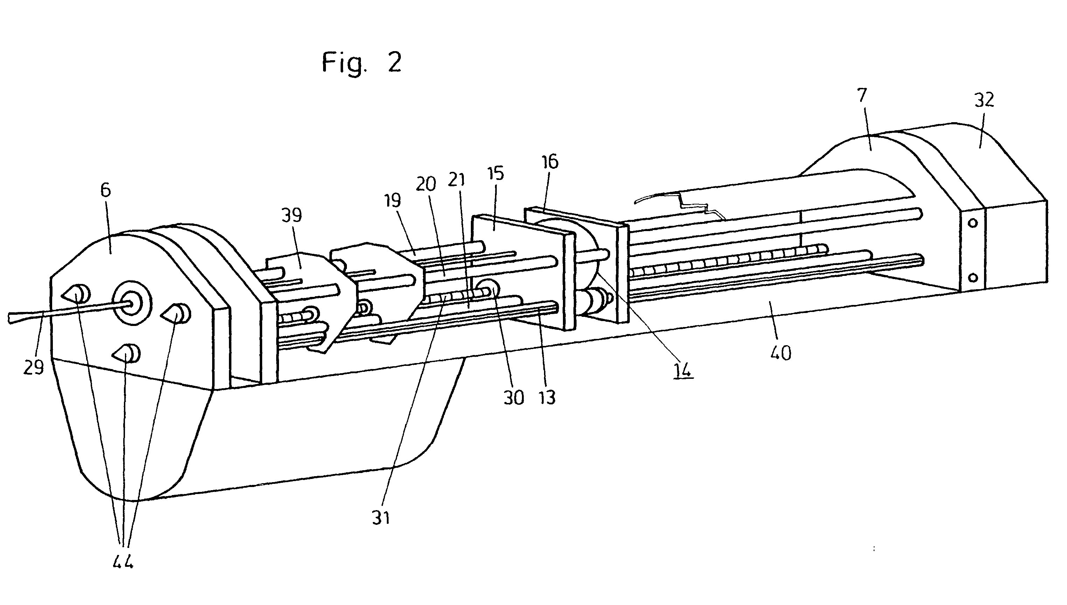

Fig. 2 einen schematisch dargestellten geöffneten Bohraufsatz.

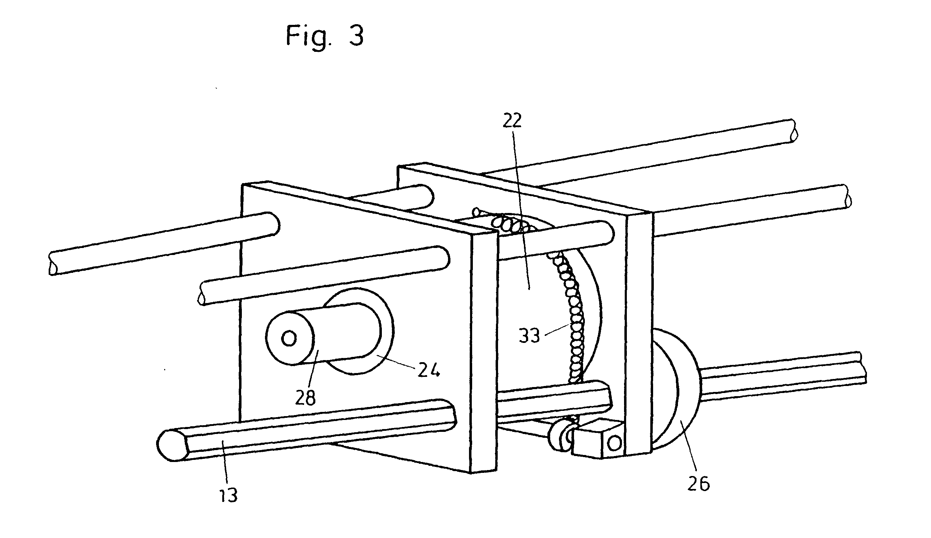

Die Fig. 3 zeigt den im Bohraufsatz verschiebbaren Schlitten, an dem die Bohrnadel befestigt ist.

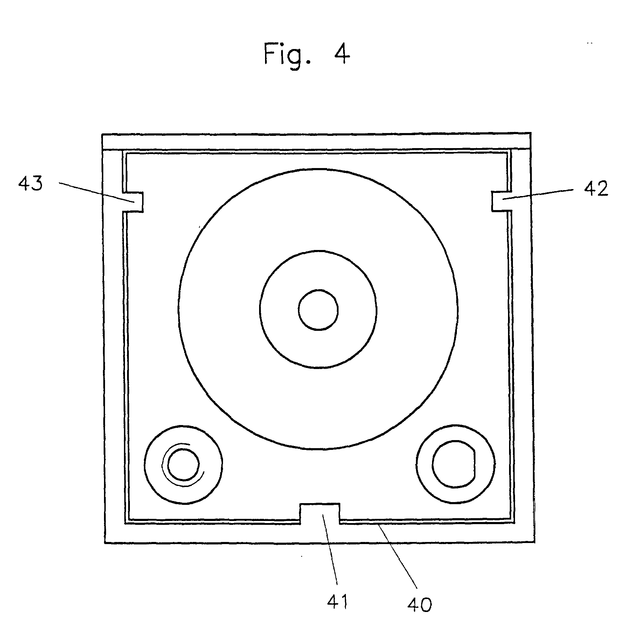

Die Fig. 4 zeigt einen Querschnit durch eine besondere Ausführungsform des rohrförmigen Körpers, bei dem die Gleitstangen durch Führungsleisten ersetzt sind.

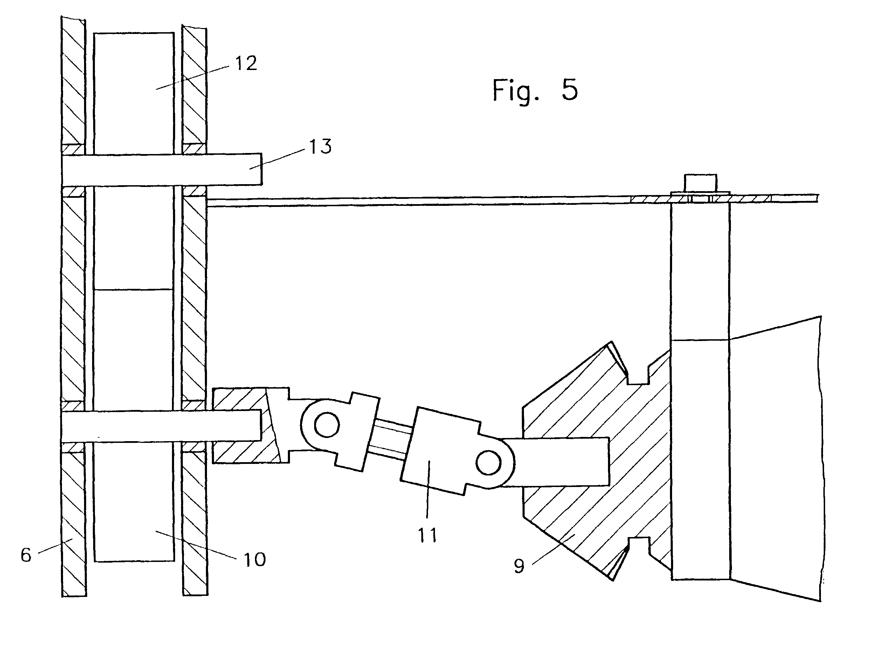

Die Fig. 5 zeigt die Kupplung zwischen Bohrmaschine und Bohraufsatz und die

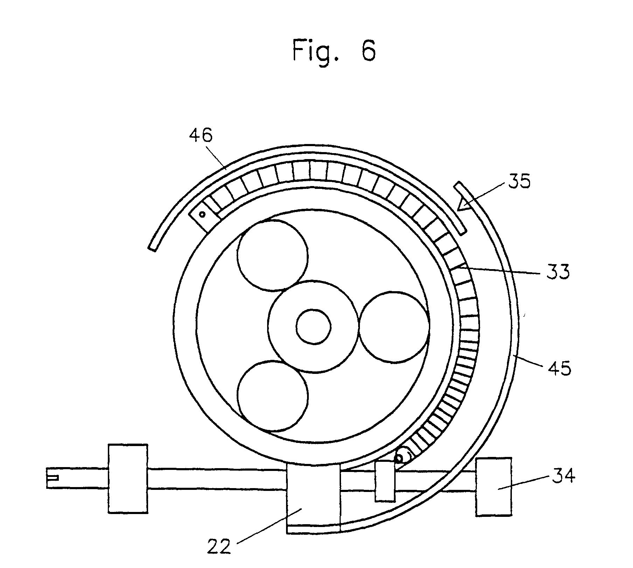

Fig. 6 das Planetengetriebe mit einem mechanischen Aufzeichnungssystem.

Die erfindungsgemäße, nachstehend detailliert beschriebene und rein mechanisch wirkende Registrierung des Bohrwiderstandes besitzt ein besseres Auflösungsvermögen, als elektrisch wirkende im Gerät eingebaute Registriereinrichtungen; es ist darauf zurückzuführen, daß dies durch einen bestimmten Energieverbrauch mit einem gewissen Dämpfungsfaktor arbeiten.

Das Ergebnis der vorstehend grundsätzlichen Prinzipien und nachfolgend detailliert beschriebenen konstruktiven Gestaltung ist ein handliches sehr gut zu handhabendes und gegenüber dem Istzustand billig herzustellendes Bohrwiderstands-Messgerät.

Diese Neuerungen sehen im Einzelnen wie folgt aus:

Für das Bohrwiderstandsmeßgerät 1 wird als Antriebsaggregat eine handelsübliche Handbohrmaschine 2 benutzt, wobei es sich sowohl um eine mit Fremdstrom betriebene oder auch um eine mit einem eigenen Akku und daher netzunabhängige Bohrmaschine handeln kann. Solche Maschinen sind heute im allgemeinen mit einer mechanischen oder elektrischen Einstellvorrichtung für verschiedene Drehzahlen, wie auch mit einem Umschalter für Rechts- oder Linkslauf ausgerüstet. Der vorgeschlagenen Anwendung kommt entgegen, daß die Hersteller solcher elektrischer Bohrmaschinen bestrebt sind, ihren Produkten nahezu gleiche Abmessungen zu geben, um beispielsweise gleiche Akkus oder Vorsatzgeräte zu verwenden, oder einen zusätzlichen zweiten Handgriff anbringen zu können. Der Bohraufsatz 3 ist daher äußerlich so ausgebildet, daß er an eine große Auswahl verschiedener Bohrmaschinen formschlüssig leicht befestigt werden kann. Wo dies nicht möglich ist, wird ein Adapter 4 verwendet, dessen Oberseite für eine formschlüssige Verbindung mit dem Bohraufsatz 3, dessen Unterseite für eine formschlüssige Verbindung mit der jeweils verwendeten Bohrmaschine 2 ausgebildet ist.

Als Antriebsaggregat kann z. B. auch ein Elektromotor oder ein pneumatisch/ hydraulisch betriebener Motor eingesetzt werden.

Der Bohraufsatz 3 besteht aus einem rohrförmigen Körper 5, der nach der Arbeitsseite hin mit einem Frontstück 6, nach der anderen Seite ein Verschlußteil 7 besitzt, die durch Verschrauben, Verkleben, Verschweißen oder einer sonstigen Verbindungsart zu einem fest miteinander verbundenen Gehäuse zusammengefügt sind.

Die Übertragung der Energie (des Drehmoments) von der Bohrmaschine 2 in den Bohraufsatz 3 erfolgt durch eine Profilwelle 8, die ein vieleckiges, vorzugsweise dreieckiges Profil besitzt und in dem Bohrfutter 9 der Bohrmaschine 2 eingespannt wird.

Diese Profilwelle wird in ein Kupplungsstück gesteckt, wodurch die Drehung von der Bohrmaschine 2 auf ein im Frontstück 6 gelagertes Abtriebsrad 12 übertragen wird. Von diesem wird über einen entweder als Zahnrad/Zahnrad oder Zahnriementrieb oder Kettenantrieb oder Keilriementrieb ausgebildeter Übertragungsmechanismus die einerseits im Frontstück 6 und anderseits im Verschlußteil 7 gelagerte Hauptantriebswelle 13 angetrieben. Diese ist entweder durch mindestens eine ebene Fläche oder durch eine Nut so ausgebildet, daß ihre Drehung auf ein gleichzeitig in Längsrichtung verschiebbares Antriebszahnrad 26 übertragen werden kann.

Im rohrförmigen Körper 5 ist ein in ihm gleitender Schlitten 14 angeordnet. Er besteht aus der vorderen Platte 15 und der hinteren Platte 16, die durch nicht dargestellte Abstandshalter mit dem Schlittengehäuse verschraubt sind. Die Abstandshalter können als Rohre ausgeführt sein und dienen so als Gleitbuchsen für den Schlitten mit denen er auf den Gleitstangen 19, 20, 21 nach vorne oder hinten bewegt werden kann.

Auf Gleitstangen 19, 20, 21 kann verzichtet werden, wenn der rohrförmige Körper 5 so ausgebildet wird, daß der Schlitten 14 auf der unteren Fläche 40 gleitet und durch Führungsleisten 41, 42,43 geführt wird, wozu die Platten 15, 16 entsprechend geformte Ausformungen besitzen. Der rohrförmige Körper wird für eine solche Ausführungsgestaltung zweckmäßig aus einem Kunststoff oder Aluminiumprofil gefertigt.

Ein Planetengetriebe 22 ist mit seiner Antriebswelle in der hinteren Platte 16 und mit seiner Abtriebswelle 24 in der vorderen Platte gelagert. Auf der nach hinten aus dem Schlitten herausragenden Antriebswelle ist ein - nicht dargestelltes - Zahnrad befestigt, das mit einem in der hinteren Platte gelagertem Antriebszahnrad 26 kämmt. Es besitzt einen Lagerzapfen, der eine dem Profil der Hauptantriebswelle 13 entsprechende Formbohrung besitzt. Das Antriebszahnrad 26 wird somit auf der Hin- und Herbewegung des Schlittens auf der Hauptantriebswelle 13 mitbewegt und durch sie in Drehung versetzt. Durch verschiedene Durchmesser von Zahnrad und Antriebszahnrad 26 kann ein Getriebeeffekt realisiert werden.

Auf der Abtriebswelle 24 des Planetengetriebes ist eine Befestigungsvorrichtung 28 für die kraftschlüssige Aufnahme der Bohrnadel 29 vorgesehen, die bevorzugt als Dreibackenfutter oder Klemmfutter ausgebildet sein kann.

Die Hin- und Herbewegung des Schlittens erfolgt durch eine fest im Gehäuse des Schlittens 14 eingesetzte Schnecke 30 auf welche die Gewindespindel 31 wirkt; diese wiederum ist im Frontstück 6 und im Verschlußstück 7 gelagert und wird über ein Wechselgetriebe 32 in Drehung versetzt, das seinerseits an der Außenseite des Verschlußstückes 7 angeordnet, von der Hauptantriebswelle 13 angetrieben wird. Das Wechselgetriebe 32 ist so ausgebildet, daß verschiedenartig gruppierte Zahnräder in Kassetten angeordnet sind, die wahlweise in das Getriebegehäuse eingesetzt werden können und dadurch das Drehzahlverhältnis zwischen der Hauptantriebswelle 13 und der Gewindewelle 31 beliebig verändert werden kann.

Das Gehäuse des im Schlitten 14 angebrachten Planetengetriebes 22 ist nicht starr mit dem Schlitten verbunden, sondern kann durch entsprechende Lagerung sich zwischen den Platten 15 und 16 frei pendelnd bewegen. Das auf dem Gehäuse wirkende Gegendrehmoment vom Antrieb der Bohrnadel wird von einer Zug- oder Druckfeder 33 abgefangen, die einmal am Gehäuse des Planetengetriebes 22 zum anderen am Schlitten 14 über einen die Federspannung einstellbarer Schraubmechanik 34 befestigt ist. Die Feder 33 kann durch einen geeigneten Mechanismus in Ihrer Federkraft verändert werden. Damit kann das Gerät auf unterschiedlich harte Materialien eingestellt werden. Am Gehäuse des Planetengetriebes 22 ist ein Schreibmechanismus 34 mit einem Schreibstift 35 befestigt, der die Schwankungen des Gegendrehmomentes auf einen Registrierpapierstreifen 36 aufzeichnet. Dieser wird durch ein Klappfenster 37 an der Oberseite des Bohraufsatzes 3 in diesen eingelegt und durch eine geeignete Klemmvorrichtung - nicht dargestellt -, gegen Verschieben gesichert.

Dadurch, daß der Schreibstift 35 mit dem Schlitten gemäß dem Eindringen der Bohrnadel 29 verschoben wird, entsteht eine Aufzeichnung, die für jede Stelle des Bohrfortschrittes den jeweils benötigten Wert des Drehmomentes für das Bohren registriert; sie ist zugleich ein Maß für die jeweilige Dichte des zu untersuchenden Prüflings.

Anstelle oder auch zusätzlich zu einer mechanisch aufgenommenen Kurve kann auch eine elektronische Aufzeichnung und/oder Speicherung gleichzeitig vorgenommen werden. Zu diesem Zwecke erhielte die Gewindespindel 31 am Wechselgetriebe 32 einen an und für sich bekannten Signalgeber, über den der Bohrfortschritt ermittelt wird; das erforderliche Drehmoment kann über einen Impulsgeber am Planetengetriebe 22 oder an geeigneter Stelle in der Lagerung der Hauptantriebswelle 13 oder über die Stromaufnahme der Bohrmaschine 2, in an und für sich bekannter Art und Weise ermittelt und aufgezeichnet werden.

Um die Vorrichtung gegen Beschädigung zu schützen, sind in an und für sich bekannter Weise Scherstifte und/oder Rutschkupplungen vorgesehen.

Am Frontstück sind Spitzen 44 angebracht, um dem Gerät durch Andrücken an das Untersuchungsobjekt einen festen Halt zu geben.

### Bezugszeichenliste:

- 1.: Bohrwiderstandsmeßgerät
- 2.: Handbohrmaschine - elektrisch/Akku betrieben
- 3.: Bohraufsatz
- 4.: Adapter
- 5.: rohrförmiger Körper
- 6.: Frontstück
- 7.: Verschlußteil
- 8.: Profilwelle
- 9.: Bohrfutter der Bohrmaschine
- 10.: Antriebszahnrad
- 11.: Kupplungsstück
- 12.: Äbtriebsrad
- 13.: Hauptantriebswelle
- 14.: Schlitten
- 15.: vordere Platte
- 16.: hintere Platte
- 19.: Gleitstangen
- 20.: Gleitstangen
- 21.: Gleitstangen
- 22.: Planetengetriebe
- 24.: Abtriebswelle
- 26.: Antriebszahnrad
- 28.: Befestigungsvorrichtung für Bohrnadel
- 29.: Bohrnadel
- 30.: Schnecke
- 31.: Gewindespindel
- 32.: Wechselgetriebe
- 33.: Zugfeder
- 34.: Einstellbare Schraubmechanik
- 35.: Schreibstift
- 36.: Registrierpapierstreifen
- 37.: Klappfenster
- 39.: Abstützscheiben
- 40.: untere Fläche des rohrförmigen Körpers (5)
- 41.: Führungsleisten
- 42.: Führungsleisten
- 43.: Führungsleisten
- 44.: Spitzen
- 45.: bogenförmiger Halter
- 46.: Schreibunterlage

## Patentansprüche

1. Bohrwiderstandsmessgerät bestehend aus einem Bohraufsatz (3) und einem Antriebsaggregat (2) zur Ermittlung des inneren Zustands und/oder der Dichte an Bäumen oder hölzernen Bauelementen mit einer dünnen ein oder mehrmals abgestützten Bohrnadel (29) und mit Mitteln zum Aufzeichnen und/oder Mitteln zum elektronischen Speichern des über die Bohrnadel wirkenden Drehmoments (Bohrwiderstand) und der Eindringtiefe der Bohrnadel, wobei das Antriebsaggregat, eine im Bohraufsatz (3) drehbar gelagerte Hauptantriebswelle (13) antreibt, wobei die Hauptantriebswelle (13)derart ausgebildet ist, daß einerseits ein auf ihr in Längsrichtung verschiebbares Antriebszahnrad (26) mit ihr in Drehung versetzt wird und über ein Planetengetriebe (22) die in einem Schlitten (14) drehbar gelagerte Bohrnadel (29) dreht und andererseits über ein Wechselgetriebe (32) eine Gewindespindel (31) antreibt, welche im Zusammenwirken mit einer fest im Schlitten (14) eingesetzten Schnecke (30) eine Hin- oder Herbewegung des Schlittens (14) bewirkt, **dadurch gekennzeichnet, daß** der Bohraufsatz (3) mit dem Antriebsaggregat (2) an dessen Oberseite zu einer Geräteeinheit verbunden wird, wobei das Antriebsaggregat (2) über ein Kupplungsstück (11) mit einem Übertragungsmechanismus an der Frontseite 6 des Bohraufsatzes (3) angekoppelt ist, und daß die Gesamtlänge des Bohrwiderstandsmeßgeräts die Länge des Bohraufsatzes (3) nicht oder nur geringfügig überschreitet.

2. Bohrwiderstandsmeßgerät nach Anspruch 1, **dadurch gekennzeichnet, daß** der Bohraufsatz (3) aus einem rohrförmigen Körper (5) besteht, der nach der Arbeitsseite hin ein Frontstück (6), nach der Gegenseite hin ein Verschlußteil (7) besitzt, welche durch Verschrauben, Verkleben, Verschweißen oder einer adäquaten Verbindungsart zu einem fest miteinander verbundenem Gehäuse zusammengefügt sind.

3. Bohrwiderstandsmeßgerät nach Anspruch 1 oder 2, **dadurch gekennzeichnet, daß** zur Übertragung der Energie von dem Antriebsaggregat (2) in den Bohraufsatz (3) eine Profilwelle (8) Anwendung findet, die ein vieleckiges, vorzugsweise ein dreieckiges Profil, besitzt, daß im Frontstück (6) ein Antriebsrad (12) gelagert ist, das ein Kupplungsstück (11) zur Aufnahme der Profilwelle (8) besitzt, daß die Hauptantriebswelle (13) einerseits im Frontstück (6) und anderseits im Verschlußteil (7) drehbar gelagert und mit einem solchen Profil an und für sich bekannter Art derart ausgebildet ist, daß ein auf ihr in Längsrichtung verschiebbares Zahnrad (26) mit ihr in Drehung versetzt wird, und daß der Übertragungsmechanismus, wie beispielsweise ein Zahnrad-Zahnrad-Trieb oder Zahnriementrieb oder Kettenantrieb die Drehbewegung von Antriebsrad (12) auf die Hauptantriebswelle (13) überträgt.

4. Bohrwiderstandsmeßgerät nach einem der Ansprüche 1 bis 3, **gekennzeichnet durch** einen in einem rohrförmigen Körper (5) gleitenden Schlitten (14), der aus einer vorderen Platte (15) und einer hinteren Platte (16) und diese auf Abstand haltenden Abstandhalter besteht, **durch** aus Rohren gebildete und damit als Gleitbuchsen für den Schlitten (14) dienende Abstandhalter,und **durch** Gleitstangen (19, 20, 21), die im Frontstück (6) und im Verschlußteil (7) verankert sind.

5. Bohrwiderstandsmeßgerät nach einem der Ansprüche 1 bis 3, **gekennzeichnet durch** einen aus Kunststoff oder Aluminiumprofil gefertigten rohrförmigen Körper (5), auf dessen innerer unterer Fläche (40) ein Schlitten (14) gleitet und **durch** Leisten (41, 42, 43) geführt wird.

6. Bohrwiderstandsmeßgerät nach einem der Ansprüche 1 bis 5, **gekennzeichnet durch** ein mit seiner Antriebswelle in der hinteren Platte (16) und mit seiner Abtriebswelle (24) in der vorderen Platte (15) gelagertes Planetengetriebe (22), einer auf der Abtriebswelle (24) für die kraftschlüssige Aufnahme der Bohrnadel (29) angeordneten Befestigungsvorrichtung (28), einem auf der nach hinten aus dem Schlitten herausragenden Antriebswelle befestigtem Zahnrad, das mit einem in der hinteren Platte (16) gelagertem Antriebszahnrad (26) kämmt, eine solche Ausgestaltung des Zahnrades, daß die **durch** seine Mittelbohrung geführte Hauptantriebswelle (13) es während der Hin- und Herbewegung des Schlittens (14) in Drehung versetzt, eine solche Veränderung der Durchmesser von Zahnrad und Antriebszahnrad (26) bei gleichbleibendem Achsabstand, daß ein Getriebeeffekt erreicht wird, ein oder mehrere Abstützscheiben (39), die aus dünnem Blech oder Kunststoffplatten bestehen, vor dem Schlitten (14) in dessen Gleitbahn auf den Gleitstangen (19, 20, 21) angeordnet sind und in der Bohrachse eine Bohrung besitzen, **durch** welche die Bohrnadel (29) geführt wird, und beim Vorschub des Schlittens zu einem dünnen Paket zusammengeschoben werden, bzw. beim Rückwärtsfahren des Schlittens **durch** dünne Kunststoffschnüre wieder in die Ausgangsposition gezogen werden.

7. Bohrwiderstandsmeßgerät nach Anspruch 1 oder 6, **dadurch gekennzeichnet, daß** das Wechselgetriebe (32) so ausgebildet ist, daß verschiedenartig gruppierte und in Kassetten angeordnete Zahnradsätze zur Verstärkung der Drehzahlverhältnisse leicht auswechselbar sind.

8. Bohrwiderstandsmeßgerät nach einem der Ansprüche 1,6 oder 7, **gekennzeichnet durch**: eine Aufzeichnungsvorrichtung für die den unterschiedlich auftretenden Bohrwiderständen entsprechenden Schwankungen des Gehäuses des Planetengetriebes (22) mittels den konstruktiven Ausbildungen: das Planetengetriebe ist mit seiner Antriebswelle und seiner Abtriebswelle (24) in der vorderen und hinteren Platte des Schlittens (14) pendelnd gelagert, das Gehäuse des Planetengetriebes (22) ist über eine Zugfeder (33) mit einer am Schlitten fest installierten Federspannvorrichtung (34) verbunden, am Gehäuse des Planetengetriebes (22) ist über einen bogenförmigen Halter (45) ein Schreibstift (35) so angebracht, daß seine Pendelbewegungen auf eine Schreibunterlage (46) aufgezeichnet werden.

9. Bohrwiderstandsmeßgerät nach einem der Ansprüche 1, 6, 7 oder 8, **dadurch gekennzeichnet, daß** an der Oberseite des Bohraufsatzes (3) ein Klappfenster (37) vorgesehen ist und daß dicht unter der Oberseite eine Schreibunterlage (46) für die Aufnahme eines Aufzeichnungsgerätes über die gesamte Länge des Schlittenweges vorgesehen ist.

10. Bohrwiderstandsmeßgerät, nach einem der Ansprüche 1,8 oder 9, **dadurch gekennzeichnet, daß** der Aufzeichnungsträger aus einem auf Länge vorgeschnittenen Blatt oder Karte besteht oder von einer Rolle gezogen wird.

11. Bohrwiderstandsmeßgerät nach einem der Ansprüche 1 bis 6, **dadurch gekennzeichnet, daß** in an und für sich bekannter Weise zum Schütze des Gerätes bei Blockierung der Bohrnadel (29) beim Prüfvorgang an bewegten Verbindungsstellen wie Zahnradbefestigungen, Kupplungen und ähnlichen Konstruktionselementen Scherstifte und/oder Rutschkupplungen an Getrieben vorgesehen werden.

12. Bohrwiderstandsmeßgerät nach einem der Ansprüche 1 bis 11, **dadurch gekennzeichnet, daß** das Antriebsaggregat eine elektrisch betriebene Handbohrmaschine (2) ist.

## Claims

1. Device for measuring drilling resistance, consisting of a drilling attachment (3) and a drive unit (2), to determine the interior state and/or density of trees or wooden structural elements by means of a thin drilling needle (29) supported once or more than once, and with means for recording, and/or averaging for electronic storage, the torque (drilling resistance) acting through the drilling needle and the depth of penetration of the drilling needle, with the drive unit driving a main drive shaft (13) supported in the drilling attachment (3) so as to be able to rotate, which main drive shaft (13) is designed in such a way that, on the one hand, a driving gearwheel (26) lengthwise movable on it is made to turn with it, rotating by means of a planetary gear (22) the drilling needle (29) supported in a slide (14) so as to be able to rotate and, on the other hand, driving a threaded spindle (31) by means of a change gear (32), which threaded spindle, in combination with a screw (30) fixed in the slide (14), causes the slide (14) to move back and forth; with the drilling attachment (3) connected to the top of the drive unit (2) so as to constitute a unit, with the drive unit (2) coupled to the front (6) of the drilling attachment (3) by a coupling section (11) with a transmission mechanism, and with the overall length of the device for measuring drilling resistance exceeding the length of the drilling attachment (3) not at all or only slightly.

2. Device measuring drilling resistance as claimed in Claim 1 above, with the drilling attachment (3) consisting of a tubular body (5) whose working side carries a front piece (6), and whose opposite side carries a closing piece (7), which are joined into a firmly interconnected housing by bolting, bonding, welding, or an adequate means of joining.

3. Device measuring drilling resistance as claimed in Claims 1 or 2 above, with a grooved shaft (8) to transmit the energy from the drive unit (2) to the drilling attachment, which grooved shaft has a polygonal, preferably triangular, profile, and with a drive wheel (12) supported in the front piece (6) which has a coupling piece (11) to accommodate the grooved shaft (8), where the main drive shaft (13) is pivoted on one side in the front piece (6) and on the other side in the closing piece (7) and is designed with a profile of a familiar type in such a way that a gearwheel (26) lengthwise movable on it is made to rotate with it, and the transmission mechanism, such as gearwheel-gearwheel drive or a toothed belt drive or chain drive, transmits the rotation from the drive wheel (12) to the main drive shaft (13).

4. Device measuring drilling resistance as claimed in any of the Claims 1 to 3 above with a slide (14) sliding in a tubular body (5), which slide (14) consists of a front plate (15) and a rear plate (16) and a spacer keeping a distance between these, with spacers made up of tubes and thus serving as slide bushes for the slide (14), and with slide bars (19, 20, 21) anchored in the front piece (6) and the closing piece (7).

5. Device measuring drilling resistance as claimed in any of the Claims 1 to 3 above, with a tubular body (5) made out of a polymer or aluminum profile on whose inner bottom surface (40) a slide (14) glides guided by strips (41, 42, 43).

6. Device measuring drilling resistance as claimed in any of the Claims 1 to 5 above, with a planetary gear (22) whose drive shaft is supported in the rear plate (16), and whose driven shaft (24) is supported in the front plate (15), with an attachment device (28) arranged on the driven shaft (24) for the non-positive accommodation of the drilling needle (29), with a gearwheel attached to the drive shaft protruding from the rear end of the slide, which gearwheel meshes with a drive gearwheel (26) supported in the rear plate, which gearwheel is designed in such a way that the main drive shaft (13) run through its center bore turns it while the slide (14) moves back and forth, with such change in the diameters of the gearwheel and the drive gearwheel (26), with the axial distance remaining unchanged, that a gear train effect is achieved, with one or more support disks (39) out of thin sheet metal or polymer plates arranged in front of the slide (14) in its slideway on the slide bars (19, 20, 21) and with a bore in the drilling axis through which the drilling needle (29) is run, and which are pushed together into a thin package during the forward movement of the slide and are pulled back into the initial position by thin polymer threads when the slide moves backward, respectively.

7. Device measuring drilling resistance as claimed in Claims 1 or 6 above, with the change gear (32) designed in such a way that gear sets grouped in various ways and arranged in cassettes can be easily exchanged to enhance the speed ratios.

8. Device measuring drilling resistance as claimed in any of the Claims 1, 6 or 7 above, with a device for recording the oscillations of the housing of the planetary gear (22) as a function of the different drilling resistances encountered, with the following design features: The planetary gear has its drive shaft and its driven shaft (24) supported in the front and rear plates of the slide (14) so as to allow oscillating movements; the housing of the planetary gear (22) is connected by means of a tension spring (33) to a spring tensioning device (34) firmly attached to the slide, and a stylus (35) is attached to the housing of the planetary gear (22) by means of a curved support (45) in such a way that its oscillating movements are recorded on a writing pad (46).

9. Device measuring drilling resistance as claimed in any of the Claims 1, 6, 7 or 8 above, with the top of the drilling attachment (3) having a top-hung window (37), and with a writing pad (46) right below the top for accepting a recording device over the entire length of the guideway of the slide.

10. Device measuring drilling resistance as claimed in any of the Claims 1, 8 or 9 above, with the recording medium being a sheet or card precut to the proper length or pulled off a roll.

11. Device measuring drilling resistance as claimed in any of the Claims 1 to 6 above, with shear pins in moved connections, such as gearwheel supports, couplings and similar design elements, and/or clutches in gear trains, to protect the unit in a familiar way in case of the drilling needle (29) locking during the inspection process.

12. Device measuring drilling resistance as claimed in any of the Claims 1 to 11 above, with an electrically driven hand drill (2) as the drive unit.

## Revendications

1. Appareil de mesure de la résistance de perçages comprenant un accessoire de perçage (3) et une unité d'entraînement (2) pour déterminer la résistance interne et/ou la densité d'arbres ou d'éléments de construction en bois, comportant une mèche (29) mince ou à plusieurs appuis et des moyens pour enregistrer et/ou des moyens pour mettre en mémoire électroniquement le couple appliqué à la mèche (résistance de perçage) et la profondeur de pénétration de la mèche,
l'accessoire entraînant un arbre principal moteur (13) monté à rotation dans l'accessoire de perçage (3), l'arbre principal (13) étant réalisé pour, d'une part, entraîner en rotation une roue dentée d'entraînement (26) coulissant longitudinalement sur cet arbre, et par une transmission planétaire (22), faire tourner la mèche (29) montée à rotation dans un chariot (14), et d'autre part entraîner par une transmission alternée (32) une broche filetée (31) qui coopère avec une vis (30) montée de manière fixe dans le chariot (14) et produisant le mouvement de va et vient du chariot (14),
**caractérisé en ce que**
l'accessoire de perçage (3) est relié à l'unité d'entraînement (2) en étant monté sur le dessus de celui-ci pour former un appareil, l'unité d'entraînement (2) étant couplée par l'intermédiaire d'une pièce d'embrayage (11) à un mécanisme de transmission du côté avant (6) de l'accessoire de perçage (3), et
la longueur totale de l'appareil de mesure de la résistance de perçage ne dépasse pas ou que très légèrement la longueur de l'accessoire de perçage (3).

2. Appareil de mesure de la résistance de perçages selon la revendication 1,
**caractérisé en ce que**
l'accessoire de perçage (3) se compose d'un corps tubulaire (5) qui possède, du côté de travail, une pièce frontale (6) et du côté opposé, une pièce de fermeture (7), ces pièces étant reliées par vissage, collage, soudage ou autres modes de liaison appropriés pour former un boîtier assemblé solidairement.

3. Appareil de mesure de la résistance de perçages selon la revendication 1 ou 2,
**caractérisé en ce qu'**
un arbre profilé (8) pour transmettre l'énergie motrice de l'unité d'entraînement (2) à l'accessoire de perçage (3), comporte une section polygonale, de préférence triangulaire,
la pièce frontale (6) étant munie d'une roue d'entraînement (12) possédant une pièce d'embrayage (11) pour recevoir l'arbre profilé (8),
l'arbre d'entraînement principal (13) est monté à rotation d'un côté dans la pièce frontale (6) et de l'autre dans la pièce de fermeture (7), et son profil et sa réalisation sont tels qu'une roue dentée (26), coulissante dans la direction longitudinale, puisse être mise en rotation par cette pièce, et
le mécanisme de transmission, comme par exemple l'entraînement à roue dentée ou une transmission à courroie dentée ou à chaîne, transmet le mouvement de rotation de la roue d'entraînement (12) à l'arbre d'entraînement principal (13).

4. Appareil de mesure de la résistance de perçages selon l'une des revendications 1 à 3,
**caractérisé par**
un chariot (14) glissant dans un corps tubulaire (5), ce chariot se composant d'une plaque avant (15) et d'une plaque arrière (16) avec un organe d'écartement porté par ces plaques, cet organe d'écartement étant formé de tubes et constituant ainsi des paliers de glissement pour le chariot (14), et les tiges de glissement (19, 20, 21) étant accrochées dans la pièce frontale (6) et dans la pièce de fermeture (7).

5. Appareil de mesure de la résistance de perçages selon l'une des revendications 1 à 3,
**caractérisé par**
un corps tubulaire (5) réalisé en matière plastique ou en un profil d'aluminium et dont la surface inférieure intérieure (40) reçoit en glissement un chariot (14) en étant guidé par des longerons (41, 42, 43).

6. Appareil de mesure de la résistance de perçages selon l'une des revendications 1 à 3,
**caractérisé par**
une transmission planétaire (22) dont l'arbre d'entraînement est monté dans la plaque amère (16) et dont l'arbre de sortie (24) est monté dans la plaque avant (15), un dispositif de fixation (28) monté sur l'arbre de sortie (24) pour recevoir par une liaison de force la mèche de perçage (29), une roue dentée fixée sur l'arbre d'entraînement sortant de l'arrière du chariot, cette roue engrenant avec une roue dentée d'entraînement (26) montée dans la plaque arrière (16), une réalisation de la roue dentée (14) de sorte que l'arbre d'entraînement principal (13) passant par son perçage central, le mette en rotation pendant le mouvement de va et vient du chariot (14) pour obtenir une modification du diamètre de la roue dentée et de la roue dentée d'entraînement (26) tout en conservant l'entr'axe pour avoir un effet de boîte de vitesses, un ou plusieurs disques d'appui (39) réalisés en tôle mince ou dans des plaques de matière plastique, installés devant le chariot (14) sur son chemin de glissement sur les tiges de guidage (19, 20, 21) et ayant un perçage dans l'axe de perçage qui guide la mèche de perçage (29), disques qui lors de l'avance du chariot sont regroupés sous la forme d'un paquet mince et lors du recul du chariot elles sont de nouveau tirées en position de sortie par un mince cordon de matière plastique.

7. Appareil de mesure de la résistance de perçages selon l'une des revendications 1 ou 6,
**caractérisé en ce que**
la transmission alternée (32) est réalisée pour permettre de changer des jeux de roues dentées groupés de manière différente et logés dans des boîtes pour amplifier le rapport des vitesses de rotation, ces boîtes étant facilement interchangeables.

8. Appareil de mesure de la résistance de perçages selon l'une des revendications 1, 6, ou 7,
**caractérisé par**
un dispositif d'enregistrement pour les différentes résistances de perçages rencontrées en fonction des oscillations du boîtier de la transmission planétaire (22) par des réalisations constructives :
la transmission planétaire est montée de façon pendulaire avec son ar-bre d'entraînement et son arbre de sortie (24) dans la plaque avant et la plaque arrière du chariot (14), le boîtier de la transmission planétaire (22) est relié par un ressort de traction (33) à un dispositif de ressort de tension (34) installé de manière fixe sur le chariot, le boîtier de la transmission planétaire (22) porte, par l'intermédiaire d'un support de forme courbe (45), une tige d'inscription (35) de façon à enregistrer ses mouvements pendulaires sur un support d'écriture (46).

9. Appareil de mesure de la résistance de perçages selon l'une des revendications 1, 6, 7, ou 8,
**caractérisé en ce que**
sur le côté supérieur de la garniture de perçage (3) il est prévu une fenêtre rabattable (37) et directement sous le côté supérieur il est prévu un support d'écriture (46) pour recevoir un appareil d'enregistrement sur toute la longueur de la course du chariot.

10. Appareil de mesure de la résistance de perçages selon l'une des revendications 1 à 8 ou 9,
**caractérisé en ce que**
le support d'enregistrement se compose d'une feuille ou d'une carte précoupée à la longueur ou tirée par un rouleau.

11. Appareil de mesure de la résistance de perçages selon l'une des revendications 1 à 6,
**caractérisé en ce que**
de manière connue en soi et pour soi, pour protéger l'appareil en cas de blocage de la mèche de perçage (29) lors d'une opération de contrôle, les points de liaison mobiles tels que les fixations des pignons dentés, les embrayages ou éléments de construction analogues comportent des goujons cisaillables et/ou des embrayages à cliquet pour les transmissions.

12. Appareil de mesure de la résistance de perçages selon l'une des revendications 1 à 11,
**caractérisé en ce que**
l'unité d'entraînement est une perceuse à main (2) à moteur électrique.
